# EUROPEAN PATENT APPLICATION

(11) **EP 4 062 900 A2**
(43) Date of publication of application: **28.09.2022**
(21) Application number: 22169439.1
(22) Date of filing: 07.03.2018
(51) Int. Cl.: A61K 8/9789, A61K 8/63, A61Q 19/00, A61Q 19/02, A61Q 19/08

(54) **SKIN CARE COSMETIC COMPOSITION**

(30) Priority: 27.04.2017 CN 201710286307
(62) Divisional of application: 18792071.5
(71) Applicant: Yang Sheng Tang (Shanghai) Cosmetic R&D Co., Ltd., Shanghai 200135 (CN)
(72) Inventor: ZHENG, Xiaowei, Shanghai, 200135 (CN); HU, Liu, Shanghai, 200135 (CN)
(74) Representative: J A Kemp LLP

(57) **Abstract**

The invention relates to a skincare cosmetic composition comprising birch sap, birch bark extract, and optional ingredients commonly used in skincare cosmetics, without water typically added as a separate component.

## Description

### Technical field

The invention relates to a skincare cosmetic composition comprising birch sap, birch bark extract, and optional ingredients commonly used in skincare cosmetics, but no water typically added as a separate component.

### Background art

Betulin, also known as birch camphor, is found in birch bark in an amount of 10-35%; it is a very valuable natural product for having anti-UV, anti-inflammatory, whitening, repairing skin and other efficacy; therefore, it is often used in the development of cosmetics, foods and drugs, for example, used in cosmetics Limi The Age of Innocenc, True Hydration 24H Gel Cream (GoodSkin Labs) and Solution; however, the efficacy of these products are not satisfactory. At present, many drugs such as safflower extract, salvia miltiorrhiza extract, ginkgo biloba extract and other conventional oral and intravenous administration routes can significantly improve skin problems, but same efficacy cannot be achieved after topical external application; it can be concluded that topical external application of drugs is affected by many factors. In general, an active substance that cannot easily penetrate into the skin will be difficult to exert corresponding efficacy. Therefore, it is a problem to be solved by those skilled in the art how to make the functional ingredients in cosmetics act on the surface of the skin or enter the epidermis or dermis according to the effectiveness of the product, and accumulate and function at the site.

Therefore, the invention attempts to provide a technique for significantly improving the transdermal absorption of betulin in a product, thereby obtaining a skincare composition which is different from the prior art and can exert better efficacy of betulin in moisturizing, whitening and antiwrinkle.

### Detailed description of the invention

An object of the invention is to provide a technique for significantly improving the transdermal absorption of betulin in a product, thereby obtaining a skincare composition which is different from the prior art, in particular, a skincare cosmetic composition comprising birch sap instead of the whole water in the formulation. The invention has surprisingly found that, after topical external application of the skincare cosmetic composition, the skincare cosmetic composition can significantly and effectively increase the transdermal absorption of betulin therein as compared with similar formulation comprising added water, thereby exerting better efficacy of betulin in moisturizing, whitening and antiwrinkle. The invention has been completed based on the above findings.

Therefore, the invention provides a skincare cosmetic composition comprising (A) 30-99wt% of birch sap, (B) 0.5 ppm-5wt% of birch bark extract, and (C) optionally, ingredients commonly used in skincare cosmetics, wherein the skincare cosmetic composition does not comprise water typically added as a separate component.

The skincare cosmetic composition according to the invention has improved moisturizing, whitening, antiwrinkle and other skincare efficacy.

The skincare cosmetic composition of the inventio does not comprises any added water, but does not exclude the inherent moisture in the birch sap used or a small or trace amount of water inevitably introduced from other ingredients.

In a preferred embodiment, the skincare cosmetic composition according to the invention does not comprise a chelating agent such as EDTA salt, sodium polyphosphate, sodium metaphosphate or gluconic acid and the like.

The invention further relates to a method for increasing the transdermal absorption of betulin, comprising adding birch sap to a skincare cosmetic composition comprising a birch bark extract but no added water, wherein the birch bark extract comprises 0.05-99.95wt% of betulin. The method can significantly increase the transdermal absorption of betulin, thereby achieving significantly improved skincare effect.

The invention also relates to use of a combination of birch sap and birch bark extract in a skincare cosmetic composition comprising no added water, wherein the birch bark extract comprises 0.05-99.95wt% of betulin.

The birch tree involved in the invention belongs to Betula Betulaceae, which may be the three varieties of Betula alba, Betula Pendula and Betula platyphylla.

The birch sap according to the invention is a colorless, transparent, fragrant and nutritious sap without precipitates or impurities which is collected by drilling holes in the birch trunk from the time of snow beginning to melt to the time of tree foliation. The birch sap is commercially available and used as it is, for example, it is commercially available from Daxinganling Chaoyue Wild Berry Development Co., Ltd.

The skincare cosmetic composition according to the invention may comprise 30-99wt%, preferably 50-95wt%, more preferably 60-90wt% of birch sap, based on the total weight of the composition.

The birch bark extract according to the invention is obtained by extracting soft bark of birch tree with one or more of solvents including, but not limited to water, methanol, ethanol, ethyl ether, petroleum ether, chloroform, and benzene. Generally, the content of betulin in the extract may vary between 0.05 and 99.95%, depending on the solvent employed and the intended use. The birch bark extract is usually in the form of a powder and is commercially available, for example, it is commercially available from Chengdu Jianteng Biotechnology Co., Ltd.

The skincare cosmetic composition according to the invention may comprise 0.5 ppm-5wt%, preferably 0.001-3wt%, more preferably 0.01-2wt% of birch bark extract, calculated in terms of betulin, based on the total weight of the composition.

The skincare cosmetic composition according to the invention also optionally comprises, as component (C), ingredients commonly used in skincare cosmetics, including a vehicle, an active ingredient, and an excipient etc. Component (C) is known in the art, and those skilled in the art can specifically select its type and amount as needed. Generally, component (C) is present in the composition of the invention in an amount of 0-70wt%, based on the total weight of the composition.

The vehicle is, for example, a diluent, a dispersant or a carrier. All of these vehicles are known in the art, and those skilled in the art can specifically select the type and amount as needed. For example, the vehicle includes, but is not limited to, ethanol, dipropylene glycol, butylene glycol, and the like. Typically, the vehicle is present in the composition of the invention in an amount of 0.5-20% based on the total weight of component (C).

The active ingredients include, for example, an emollient, a moisturizer, a skin conditioner, and the like. All of these active ingredients are known in the art, and those skilled in the art can specifically select the type and amount as needed.

For example, the emollient includes, but is not limited to, one or more of olive oil, macadamia nut oil, sweet almond oil, grape seed oil, avocado oil, corn oil, sesame oil, soybean oil, peanut oil, meadowfoam seed oil, safflower seed oil, rosa canina fruit oil, argania spinosa kernel oil, simmondsia chinensis seed oil, sunflower seed oil, mauritia flexuosa fruit oil, squalane, ethylhexyl palmitate, isopropyl myristate, hydrogenated polyisobutylene, isocetane, isododecane, diethylhexyl carbonate, dioctyl carbonate, isopropyl lauroyl sarcosinate, isononyl isononanoate, hydrogenated polydecene, triethylhexanoin, cetyl ethylhexanoate, bis-ethoxydiglycol cyclohexane 1,4-dicarboxylate, caprylic/capric triglyceride, oleyl erucate, octyldodecanol myristate, octyldodecanol, polydimethylsiloxane, octyl polymethylsiloxane, cetyl dimethicone, cyclopentadimethylsiloxane, and so on. The solid emollient according to the invention includes, but is not limited to, one or more of cetyl alcohol, stearyl alcohol, cetearyl alcohol, behenyl alcohol, squalyl alcohol, lauric acid, myristic acid, palmitic acid, stearic acid, beeswax, candelilla wax, carnauba wax, lanolin, ozokerite, jojoba seed wax, paraffin wax, microcrystalline wax, hydrogenated rice bran wax, hydrogenated cocoglycerides, glyceryl behenate/eicosadioate, myristyl myristate, bis-diglyceryl polyacyladipate-2, butyrospermum parkii (shea butter), astrocaryum murumuru seed butter. The content of the emollient in the composition is known in the art. Typically, the emollient is present in the composition of the invention in an amount of 1-50wt% based on the total weight of the component (C).

For example, the moisturizer includes, but is not limited to, one or more of glycerol, diglycerol, propylene glycol, 1,3-propanediol, 1,2-pentanediol, polyethylene glycol-8, polyethylene glycol-32, methyl gluceth-10, methyl gluceth-20, PEG/PPG-17/6 copolymer, glycereth-7, glycereth-26, glyceryl glucoside, PPG-10 methyl glucose ether, PPG-20 methyl glucose ether, PEG/PPG/polybutylene glycol-8/5/3 glycerol, sucrose, trehalose, rhamnose, mannose, raffinose, betaine, erythritol, xylitol, urea, glycereth-5 lactate, sodium hyaluronate, sodium polyglutamate, hydrolyzed sclerotium gum, pululan, tremellam, tamarindus indica seed polysaccharide, and so on. The content of the moisturizer in the composition is known in the art. Typically, the moisturizer is present in the composition of the invention in an amount of 1-30wt% based on the total weight of the component (C).

The skin conditioner may include active ingredients for moisturizing, antiwrinkle, anti-freckle, anti-acne, oil control, etc., including but not limited to one or more of kojic acid, ascorbic acid, ascorbyl glucoside, arbutin, tranexamic acid, nicotinamide, phytosterols, phytosteryl/behenyl /octyldodecyl lauroyl glutamate, phenylethyl resorcinol, turmeric extract, birch bark extract, ceramide 2, ceramide 3, acetyl phytosphingosine, resveratrol, pterocarpus marsupium bark extract, plectranthus barbatus root extract, pepper seed extract, ubiquinone, cholesterol, cholesterol stearate, ascorbyl dipalmitate, Tocopherol (vitamin E), tocopheryl acetate, alpha-bisabolol, ascorbyl tetraisopalmitate, pyridoxine dicaprylate, pyridoxine dipalmitate, retinyl palmitate, phytosteryl /octyldodecyl lauroyl glutamate, bis-behenyl/isostearyl/phytosteryl dimer dilinoleyl dilinoleate, phytosteryl macadamiate, peptides, plant extracts and the like. The content of the skin conditioner in the composition is known in the art. Typically, the skin conditioner is present in the composition of the invention in an amount of 0.01-50wt% based on the total weight of the component (C).

The excipient includes, but is not limited to, an emulsifier, a thickener, a preservative, a perfume, and the like. All of these excipient ingredients are known in the art, and those skilled in the art can specifically select the type and amount as needed.

For example, the emulsifier includes, but is not limited to, one or more of cetearyl olivate, sorbitan olivate, polysorbate-60, polysorbate-80, methyl glucose sesquistearate, PEG-20 methyl glucose sesquistearate, PEG-40 hydrogenated castor oil, PPG-26-buteth-26, PEG-4 polyglyceryl-2 stearate, PEG-60 Hydrogenated castor oil, steareth-2, steareth-21, PPG-13-decyltetradeceth-24, cetearyl glucoside, PEG-100 stearate, glyceryl stearate, glyceryl stearate SE, coco glucoside, ceteareth-25, PEG-40 stearate, polyglyceryl-3 methyl glucose distearate, glyceryl stearate citrate, polyglyceryl-10 stearate, polyglyceryl-10 myristate, polyglyceryl-10 dioleate, polyglyceryl-10 laurate, polyglyceryl-10 isostearate, polyglyceryl-10 oleate, polyglyceryl-10 diisostearate, polyglyceryl-6 laurate, polyglyceryl-6 myristate, sucrose stearate, sucrose polystearate, etc. The content of the emulsifier in the composition is known in the art. Typically, the emulsifier is present in the composition of the invention in an amount of 0.5-10wt% based on the total weight of the component (C).

For example, the thickener includes, but is not limited to, one or more of high molecular polymers such as carbomers, acrylates and derivatives thereof, xanthan gum, gum arabic, polyethylene glycol-14M, polyethylene glycol-90M, succinyl polysaccharides, hydroxyethyl cellulose, hydroxypropyl cellulose, and hydroxypropyl methyl cellulose. The content of the thickener in the composition is known in the art. Typically, the thickener is present in the composition of the invention in an amount of 0.1-10wt% based on the total weight of the component (C).

For example, the preservative includes, but is not limited to, one or more of methyl hydroxybenzoate, propyl hydroxybenzoate, phenoxyethanol, benzyl alcohol, phenylethanol, bis(hydroxymethyl)imidazolidinyl urea, potassium sorbate, sodium benzoate, chlorophenesin, sodium dehydroacetate, etc. The content of the preservative in the composition is known in the art. Typically, the preservative is present in the composition of the invention in an amount of 0.01-2wt% based on the total weight of the component (C).

The skincare cosmetic composition of the invention can be prepared by any suitable method known in the art. For example, it can be prepared by a container commonly used in the field of cosmetics, such as a dissolution tank, an emulsification pot, a disperser, a transfer pump or the like. In the preparation, watersoluble substances are introduced into an aqueous phase dissolution kettle, and oil-soluble substances are introduced into an oil phase dissolution tank, and the two tanks are heated to about 80°C, respectively; for agglomerated raw materials, they can be pre-dispersed by a disperser. After the dissolution is completed, the oil phase and the aqueous phase are transferred into an emulsification pot, and homogenized and emulsified for about 5-15 mins. After the emulsification is completed, the temperature of the bulk is reduced to room temperature, optionally a fragrance, a preservative and the like are added, and the pH of the product is adjusted as needed. The relevant tests are done before the product is packaged and shipped. Above preparation methods can be revised and adjusted according to the product form requirements, and various product forms such as cream, lotion, essence, facial cleaner can be prepared as needed.

In a preferred embodiment, the invention provides a skincare composition having following ingredients, preferably a toner, which exhibits an excellent moisturizing, whitening, antiwrinkle and other skincare efficacy.

| Ingredients | content (wt.% ) |
|---|---|
| BETULA ALBA sap | 70-90 |
| Methyl hydroxybenzoate | 0.05-0.4 |
| Birch bark extract (containing 40% betulin) | 0.00001-1 |
| Pentanediol | 1-5 |
| Glycerol | 1-10 |
| Dipropylene glycol | 0.5-5 |
| Butanediol | 1-10 |
| Citric acid | 0.01-0.5 |
| Arginine | 0.05-1 |

### Examples

The invention is further illustrated by the following examples and comparative examples, but it should be understood that these examples and comparative examples are only for the purpose of more detailed description and are not intended to limit the invention in any way.

### Example 1: Transdermal absorption of betulin in different systems

In this example, the transdermal absorption of betulin in aqueous and nonaqueous systems is tested and compared. The specific test formulation is shown in Table 1.

**Table 1**

| Ingredients | Weight | | | |
|---|---|---|---|---|
| | Formulation 1 the invention | Formulation 2 control | Formulation 3 control | Formulation 4 control |
| Purified water ( g ) | 0 | 19 | 75.9 | 94.9 |
| Birch sap^{a} ( g ) | 94 . 9 | 75.9 | 19 | 0 |
| Birch bark extract^{b} ( g ) content of botulin:0.5% | 0.1 | 0.1 | 0.1 | 0.1 |
| Pentanediol* | 5 | 5 | 5 | 5 |
| Total ( g ) | 100 | 100 | 100 | 100 |

| | | | | |
|---|---|---|---|---|
| Notes: a: the birch sap used in the invention is commercially available from Daxinganling Chaoyue Wild Berry Development Co., Ltd. b: the birch bark extract used in the invention is commercially available from Chengdu Jianteng Biotechnology Co., Ltd. *: since betulin is insoluble in water, adding a proper amount of pentanediol helps it to dissolve. | | | | |

The formulation is prepared as follows:
1. Birch bark extract and pentanediol are mixed and heated to 80°C, stirred and dissolved uniformly.
2. BETULA ALBA sap/water is heated to 80°C, and then add the mixture obtained in the above 1.
3. The resultant is cooled down to 40°C, and discharged.

Test method: a commercially obtained unhaired pig skin is cut into 2.5 cm x 2.5 cm pieces. 1 g of the formulations 1-4 are applied onto each piece of the pig skin, and placed in an incubator with 30°C constant temperature and constant relative humidity (RH) of 50 ± 5% for 24 h, respectively. Six parallel experiments are performed in each group. Then, the pig skin is rinsed, chopped, and ultrasonically extracted with methanol for 30 mins. The extracted liquids are combined and concentrated to 5.0 ml under reduced pressure, centrifuged, and the supernatant is filtered through a filter and injected into a high performance liquid chromatograph for testing, and the content of betulin is calculated by an external standard method. The test results are shown in Table 2 below.chromatography. The test is carried out in the instrument, and the content of betulin is calculated by external standard method. The test results are shown in Table 2.

**Table 2**

| Samples | | content of betulin |
|---|---|---|
| Birch bark extract(%) | | 0.50 |
| Formulation 1 the invention | content of betulin in the formulation ( ug/g sample ) | 4.95 |
| | transdermal absorption of betulin (ug) | 3.21 ±0.60 |
| Formulation 2 control | content of betulin in the formulation ( ug/g sample ) | 4.93 |
| | transdermal absorption of betulin (ug) | 0.32±0.01* |
| Formulation 3 control | content of betulin in the formulation ( ug/g sample ) | 4.98 |
| | transdermal absorption of betulin (ug) | 0.27±0.02* |
| Formulation 4 control | content of betulin in the formulation ( ug/g sample ) | 5.02 |
| | transdermal absorption of betulin (ug) | 0.01±0.01* |

| | | |
|---|---|---|
| Note*: examined by independent t, as compared with formulation 1, formulations 2-4 exhibit significantly decreased transdermal absorption of betulin (P<0.01). | | |

The results show that, as compared with the aqueous system, the system comprising birch sap but no added water according to the invention exhibits significantly increased transdermal absorption effect of betulin.

### Example 2: liquid skincare composition 1 of the invention vs. control liquid skincare compositions 1-2

The formulations of the compositions are shown in Table 3.

**Table 3**

| Phase | Components | Skincare composition 1, the invention | Control composition 1 | Control composition 2 |
|---|---|---|---|---|
| | | Weight ratio ( %) | Weight ratio ( %) | Weight ratio ( %) |
| A | BETULA ALBA sap | 90 | 0 | 90.1 |
| | Deionized water | 0 | 90 | 0 |
| | Methyl hydroxybenzoate | 0.15 | 0.15 | 0.15 |
| | Citric acid | 0.1 | 0.1 | 0.1 |
| | Hydroxyethylcellulose | 0.1 | 0.1 | 0.1 |
| | Sodium hyaluronate | 0.05 | 0.05 | 0.05 |
| | Glycerol | 4 | 4 | 4 |
| | Dipropylene glycol | 3 | 3 | 3 |
| B | Dipropylene glycol | 1 | 1 | 1 |
| | BETULA ALBA bark extract (9% botulin content) | 0. 1 | 0. 1 | 0 |
| C | Glycereth-26 | 1 | 1 | 1 |
| | Arginine | 0.2 | 0.2 | 0.2 |
| | Phenoxyethanol | 0.3 | 0.3 | 0.3 |

The compositions are prepared as follows:
1. The phase B is mixed and heated to 80°C, stirred and dissolved uniformly.
2. The raw materials in phase A are heated to 80°C, stirred and dissolved uniformly.
3. The well-dissolved phase A and phase B are mixed, cooled down to 40°C; then the raw materials of phase C are added, stirred uniformly and discharged.

The composition 1 of the invention and control compositions 1-2 prepared above are used in the experiments to test their moisturizing efficacy.

A total of 16 volunteers are recruited, and a 3 cm x 3 cm area is identified by a template on the medial skin of the right forearm of the subject, and there are three areas. Diet or strenuous activity is avoided when the test is carried out, and the forearm is exposed and placed in a test state to keep relaxed. One area is applied with the composition 1 of the invention, the second area is applied with the control composition 1, the third area is applied with the control composition 2, and the test samples are applied at a dose of (2.0 ± 0.1) mg/cm², and the subjects are asked to apply once in the morning and evening (after bathing).The moisture content of skin epidermis is measured by an instrument in 0 day before application (reference) and in 7 days and 14 days after application. The test is repeated three times for each area, and the test results are averaged. Statistical analysis is performed using SPSS16.0 software. The data are expressed as mean and standard deviation. The data before and after the application are analyzed by t test. p<0.05 is considered statistically significant. The test results are shown in Table 4.

**Table 4**

| Days | Composition 1, the invention | Control composition 1 | Control composition 2 |
|---|---|---|---|
| 0 | 24.31% | 25.76% | 24.83% |
| 7 | 47.12%^{abc} | 32.15%^{ac} | 38.52%^{ab} |
| 14 | 46.45%^{abc} | 33.47%^{ac} | 36.78%^{ab} |

| | | | |
|---|---|---|---|
| Notes: a: as compared with the reference, there is significant difference (P<0.01) b: as compared with control 1, there is significant difference (P<0.01) c: as compared with control 2, there is significant difference (P<0.01) | | | |

The results show that, after continuous application for 14 days, the skin moisturizing performance of the composition 1 of the invention is improved by 91%, the skin moisturizing performance of the control composition 1 is improved by 30%, and the skin moisturizing performance of the control composition 2 is improved by 48%. Therefore, the composition 1 of the invention has a better moisturizing effect than the control compositions 1-2, showing that birch sap and BETULA ALBA bark extract exhibit a certain synergistic effect.

### Example 3: skincare lotion composition 2 of the invention vs. Control lotion composition 3

The formulations of the compositions are shown in Table 5.

**Table 5**

| Phase | Components | Composition 2, the invention | Control composition 3 |
|---|---|---|---|
| | | Weight ratio ( %) | Weight ratio ( %) |
| A | BETULA ALBA sap | 74.8 | 0 |
| | Deionized water | 0 | 74.8 |
| | Methyl hydroxybenzoate | 0.2 | 0.2 |
| | Citric acid | 0.1 | 0.1 |
| | Xanthan gum | 0.2 | 0.2 |
| | Sodium hyaluronate | 0.05 | 0.05 |
| | Glycerol | 6 | 6 |
| | 1,3-propanediol | 4 | 4 |
| | methyl gluceth-20 | 2 | 2 |
| | Acrylates/C10-30 alkyl acrylate crosspolymer | 0.2 | 0.2 |
| B | Propyl hydroxybenzoate | 0.1 | 0.1 |
| | Cetearyl glucoside, cetyl alcohol,stearyl alcohol | 1 | 1 |
| | Caprylic/capric triglyceride | 6 | 6 |
| | Astrocaryum murumuru seed butter | 0.5 | 0.5 |
| | Cetyl alcohol | 1 | 1 |
| | Cyclopentadimethylsiloxane | 1 | 1 |
| C | BETULA ALBA bark extract ( containing 40% betulin) | 0.5 | 0.5 |
| | Isopropyl lauroyl sarcosinate | 2 | 2 |
| D | Arginine | 0.35 | 0.35 |
| | Phenoxyethanol | 0.4 | 0.4 |

The compositions are prepared as follows:
1. Glycerol, xanthan gum, sodium hyaluronate and acrylates/C10-30 alkyl acrylate crosspolymer in phase A are mixed with stirring uniformly in a container in advance; the other raw materials in phase A are heated to 80°C, and the above pre-dispersed raw materials are added, dissolved and mixed uniformly.
2. The raw materials of phase B are heated to 80°C, dissolved and mixed uniformly.
3. The raw materials of phase C are heated to 80°C, dissolved and mixed uniformly.
4. Phase A, Phase B and Phase C are added to an emulsification pot, held at 80°C, homoemulsified for 5 mins at a speed of 3000 rpm, after the completion of the emulsification, arginine in phase D is added to adjust the pH.
5. The resultant is stirred and cooled down to 40°C, phenoxyethanol in phase D is added, stirred uniformly, and discharged.

The contents of betulin in the birch bark extract, the composition 2 of the invention, and the control composition 3 are measured in accordance with the test method in Example 1. The test results are 40.2%, 0.201 g/100 g sample and 0.198 g/100 g sample, respectively.

The compositions prepared above are used for experiments to compare the whitening efficacy.

34 volunteers, 16-18 ones in each group, having a skin blackness value of > 150, are chosen, sex randomization, and each group has a balanced age distribution. The medial skin of the forearm of the left arm is selected as test site, and the right arm is a control group. The LAB value of the medial skin of the forearm of the left arm before and after application of samples is obtained by a skin color test probe (Colorimeter CL400). The data are collected "before application", "in 4 weeks after application", and "in 8 weeks after application", respectively. The data are statistically analyzed by SPSS to evaluate the long-term whitening efficacy of the two samples. The data before and after the application are analyzed by t test, p<0.05 is considered statistically significant. The test results are shown in Table 6.

**Table 6: Average improvement of L value for composition 2 of the invention and control composition 3**

| week | Composition of the invention | Control composition 3 |
|---|---|---|
| 0 | 61.34 | 58.78 |
| 4 | 72.23 ^{ab} | 65.34 |
| 8 | 75.45 ^{ab} | 66.87 |

| | | |
|---|---|---|
| Notes: a: as compared with the reference, there is significant difference (P<0.01) b: as compared with control composition 2, there is significant difference (P<0.01) | | |

The results show that, the skincare lotion composition 2 of the invention has better effect of improving skin color (whitening) than control composition 3.

### Example 4: The cream composition 3 of the invention vs. control cream composition 4

The formulations of the compositions are shown in Table 7.

**Table 7**

| Phase | Components | Composition 3, the invention | Control composition 4 |
|---|---|---|---|
| | | Weight (%) | Weight %) |
| A | BETULA ALBA sap | 67.85 | 0 |
| | Deionized water | 0 | 67.85 |
| | Methyl hydroxybenzoate | 0.2 | 0.2 |
| | Citric acid | 0.1 | 0.1 |
| | Xanthan gum | 0.2 | 0.2 |
| | Sodium hyaluronate | 0.05 | 0.05 |
| | Glycerol | 5 | 5 |
| | PPG-10 methyl glucose ether | 2 | 2 |
| | Acrylates/C10-30 alkyl acrylate crosspolymer | 0.2 | 0.2 |
| B | Propyl hydroxybenzoate | 0.1 | 0.1 |
| | Polysorbate-60 | 0.5 | 0.5 |
| | Cetearyl glucoside, sorbitan olivate, cetearyl alcohol | 3 | 3 |
| | Caprylic/capric triglyceride | 6 | 6 |
| | squalane | 4 | 4 |
| | Butyrospermum Parkii (Shea Butter) | 3 | 3 |
| | Cetearyl alcohol | 3 | 3 |
| | Polydimethylsiloxane | 2 | 2 |
| C | BETULA ALBA bark extract (containing 80% betulin) | 1 | 1 |
| | Isopropyl lauroyl sarcosinate | 4 | 4 |
| D | Arginine | 0.3 | 0.3 |
| | Phenoxyethanol | 0.4 | 0.4 |

The compositions are prepared as follows:
1. Glycerol, xanthan gum, sodium hyaluronate and acrylates/C10-30 alkyl acrylate crosspolymer in phase A are mixed with stirring uniformly in a container in advance; the other raw materials in phase A are heated to 80°C, and the above pre-dispersed raw materials are added, dissolved and mixed uniformly.
2. The raw materials of phase B are heated to 80°C, dissolved and mixed uniformly.
3. The raw materials of phase C are heated to 80°C, dissolved and mixed uniformly.
4. Phase A, Phase B and Phase C are added to an emulsification pot, held at 80°C, homoemulsified for 5 mins at a speed of 3000 rpm, after the completion of the emulsification, arginine in phase D is added to adjust the pH.
5. The resultant is stirred and cooled down to 40°C, phenoxyethanol in phase D is added, stirred uniformly, and discharged.

The contents of betulin in the birch bark extract, the composition 3 of the invention, and the control composition 4 are measured in accordance with the test method in Example 1. The test results are 80.1%, 0.802 g/100 g sample and 0.799 g/100 g sample, respectively.

The compositions prepared above are used for full face contrast experiments to evaluate anti-aging efficacy.

A total of 70 volunteers, 35 volunteers in each group, age of > 40 years old, are chosen, female, and each group has a balanced age distribution. One group uses composition 3 and one group uses control 4. According to the dose of 2mg/cm², the full face usage is about 0.3g. Volunteers, according to the usual habits of applying cream cosmetics, take suitable amount of samples for gentle massage on the face until completely absorbed, twice a day, the time interval of two applications is at least 8 h, and it is ensured that the product stay on the skin for at least 6 h. In 1 month and 2 months after application of the products, the face is photographed separately, and then the elasticity value R2 of the facial skin cheek portion (Germany CK (COURAGE+KHAZAKA)) is tested. The data are statistically analyzed by SPSS to evaluate the anti-aging efficacy of the two samples. The date before and after the application are analyzed by t test, p<0.05 is considered statistically significant. The test results are shown in Table 8.

**Table 8: anti-aging efficacy of composition 3 of the invention and control composition 4**

| week | Composition 3 of the invention | Control composition 4 |
|---|---|---|
| 0 | 0.688 | 0.677 |
| 4 | 0.734 ^{ab} | 0.687 |
| 8 | 0.765^{ab} | 0.702 |

| | | |
|---|---|---|
| Notes: a: as compared with the reference, there is significant difference (P<0.01) b: as compared with control composition 4, there is significant difference (P<0.01) | | |

The results show that, the skincare cream composition 4 of the invention has better efficacy of improving skin's elasticity than control composition 4.

## Claims

1. A skincare cosmetic composition, comprising:
(A) 30-99wt% of birch sap,
(B) 0.5 ppm-5wt% of birch bark extract, calculated in terms of betulin, and
(C) optionally, ingredients commonly used in skincare cosmetics,
wherein the percentage is based on the total weight of the composition, and the skincare cosmetic composition does not comprise water typically added as a separate component.

2. The skincare cosmetic composition according to claim 1, wherein the composition does not comprise a chelating agent.

3. The skincare cosmetic composition according to claim 1 or 2, wherein the composition comprises 50-95wt% or 60-90wt% of birch sap.

4. The skincare cosmetic composition according to any of claims 1-3, wherein the composition comprises 0.001-3wt% or 0.01-2wt% of birch bark extract.

5. The skincare cosmetic composition according to any of claims 1-4, wherein the birch bark extract comprises 0.05-99.95wt% of betulin.

6. A method for increasing the transdermal absorption of betulin, comprising adding birch sap to a skincare cosmetic composition comprising birch bark extract but no added water, wherein the birch bark extract comprises 0.05-99.95wt% of betulin.

7. The method according to claim 6, wherein the composition comprises 30-99wt% of birch sap and 0.5 ppm-5wt% of birch bark extract, calculated in terms of botulin, based on the total weight of the composition.

8. The method according to claim 6 or 7, wherein the composition does not comprise a chelating agent.

9. Use of a combination of birch sap and birch bark extract in a skincare cosmetic composition comprising no added water, wherein the birch bark extract comprises 0.05-99.95wt% of betulin.

10. The use according to claim 9, wherein 30-99wt% of birch sap and 0.5 ppm-5wt% of birch bark extract, calculated in terms of botulin, are combined, based on the total weight of the composition.

11. The use according to claim 9 or 10, wherein the composition does not comprise a chelating agent.
